# EUROPEAN PATENT APPLICATION

(11) **EP 4 371 587 A1**
(43) Date of publication of application: **22.05.2024**
(21) Application number: 22842368.7
(22) Date of filing: 07.07.2022
(51) Int. Cl.: A61L 27/54, A61L 27/20, A61K 8/60, A61K 8/73, A61Q 19/08, A61M 5/28

(54) **METHOD FOR PREPARATION OF FILLER CONTAINING DNA FRACTION AND FILLER PREPARED THEREBY**

(30) Priority: 14.07.2021 KR 20210092042
(71) Applicant: Humedix Co., Ltd., Seongnam-si, Gyeonggi-do 13449 (KR)
(72) Inventor: KU, Min Kyung, Seoul 05639 (KR); KWON, Soon Min, Gyeongju-si Gyeongsangbuk-do 38068 (KR); MIN, Seon Hong, Incheon 21982 (KR); HA, Hye Ran, Ansan-si Gyeonggi-do 15541 (KR); CHAE, Seo Kwang, Anyang-si Gyeonggi-do 13961 (KR); HAN, Ga Dug, Seongnam-si Gyeonggi-do 13206 (KR); LIM, Chae Young, Hwaseong-si Gyeonggi-do 18452 (KR); WON, Chi Yeop, Seongnam-sI Gyeonggi-do 13531 (KR)
(74) Representative: Zacco Sweden AB
(86) International application number: PCT/KR2022/009839
(87) International publication number: WO 2023/287106

(57) **Abstract**

The present invention relates to a method for preparation of a filer containing a DNA fraction and a filler prepared thereby. Despite low contents of the components in the filler, the preparation method of the present invention can secure viscoelasticity in the filler as a physical support and has a high efficiency due to the advantage of preparation processes thereof. The filler prepared through the filler preparation method of the present invention is superb in terms of skin thickness improvement, skin wrinkle reduction effect, and collagen formation ability and causes low pain during injection, giving high satisfaction to patients and operators.

## Description

### Technical Field

The present disclosure relates to a method of preparing a filler containing a DNA fraction and to a filler prepared thereby.

### Background Art

The structure of soft tissue in the human body is maintained by proteins, such as collagen, elastin, etc., and an extracellular matrix containing glycosaminoglycans. Various factors cause soft tissue defects. In this case, the original form of the defective soft tissue is restored or corrected by surgically or nonsurgically injecting biological tissue or synthetic chemical compounds.

A filler injected for the restoration or correction is a medical device using the principle of autonomously maintaining the volume thereof through physical repair by being injected subcutaneously for temporary improvement, volume recovery, and the like around adult facial wrinkles. Typically, a filler is composed of a syringe filled with an injection solution and paired with an injection needle used together.

Currently available filler products are mainly hyaluronic acid-based. To increase the chemical and structural stability of hyaluronic acid and maintain its physicochemical properties in vivo for a long term, a method of stabilizing hyaluronic acid with the addition of a cross-linking agent has been used (International Publication Patent No. WO/2019/130360). However, the components of the cross-linking agent decomposed in vivo may be recognized as foreign substances, and inflammatory responses thus may occur. As a result, a step of removing the cross-linking agent through washing during the process is necessary, and examination of the residual amount of the cross-linking agent is inevitable, which is problematic for the efficiency of the preparation process.

In addition, in the case of hyaluronic acid-based fillers, gel-filled syringes are mainly sterilized by a method performed under high-temperature conditions using an autoclave and the like. However, there may be a problem in that viscoelasticity is reduced during the sterilization process. In the case of containing hyaluronic acid at high concentrations or using cross-linked hyaluronic acid to make up for the problem, there may be a problem in that patients have severe pain during the treatment, which makes patient satisfaction low and also makes the treatment inconvenient for practitioners.

On the other hand, to make up for the functional aspects of the hyaluronic acid-based fillers, research is in progress on fillers composed of polynucleotides and the like. However, during the sterilization process required to be involved during the preparation process, there is a problem in that the functionality of polynucleotides mixed in the actual preparation process is poor compared to the amount thereof due to the denaturation of polynucleotides. Alternatively, it is problematic that the amount of polynucleotide causes pain during the treatment, making patient satisfaction low.

### Disclosure

### Technical Problem

The inventors of the present disclosure have endeavored to solve problems in the related art. As a result, when using a specific preparation method in filler preparation, process advantages enable the preparation efficiency to be increased and a filler prepared thereby to obtain viscoelasticity despite the low amounts of constituting components. In addition, the inventors have proved that the filler prepared by the filler preparation method has excellent effects of improving skin thickness and reducing skin wrinkles, collagen formation ability, and the like, compared to conventionally available products while causing low pain during injection, giving high satisfaction to patients and practitioners. As a result, the inventors have completed the present disclosure.

The present disclosure provides a method of preparing a filler, which includes: a) mixing a DNA fraction with a solvent to produce a solution; b) removing bacteria from the produced solution using a sterile filter; and c) mixing hyaluronic acid and/or a pH modifier with the bacteria-removed solution.

The present disclosure provides a filler prepared by the preparation method described above.

The present disclosure provides a pre-filled syringe filled with the filler described above.

### Technical Solution

Hereinafter, the present disclosure will be described in detail. All combinations of a variety of elements disclosed herein fall within the scope of the present disclosure. In addition, the scope of the present disclosure is not deemed to be limited by the detailed description below.

The preset disclosure provides a method of preparing a filler, which includes: a) step of mixing a DNA fraction with a solvent to produce a solution; b) step of removing bacteria from the produced solution using a sterile filter; and c) step of mixing hyaluronic acid and/or a pH modifier with the bacteria-removed solution.

Each step of the preparation method will be described in detail below.

In the present disclosure, the a) mixing step is a step of mixing the DNA fraction with the solvent to produce the solution in which the DNA fraction is dissolved.

In the present disclosure, the "DNA fraction" means a chain-type polymer formed by covalently linking the 3rd carbon of a pentose constituting a nucleotide to the phosphoric acid group of another nucleotide. The nucleotide is a molecule to which one phosphoric acid, one sugar, and one base are each independently linked, and is a unit constituting a nucleic acid containing adenine (A), guanine (G), cytosine (C), thymine (T), and uracil (U).

In the present disclosure, the DNA fraction can promote the production of extracellular matrix (ECM), which is an intercellular matrix, and can activate skin healing ability in the human body. For this reason, the skin can function to restore deteriorated and weakened regeneration ability by itself, so the DNA fraction can serve to improve the functionality of the skin itself.

In the present disclosure, the DNA fraction may include the DNA fraction itself, a salt thereof, or a combination thereof. In addition, the DNA fraction may be directly synthesized or purchased from a commercially available product for use.

In the present disclosure, the DNA fraction may be polynucleotide (PN), polydeoxyribonucleotide (PDRN), or a mixture thereof, and includes fragments thereof. According to embodiments of the present disclosure, the DNA fraction may be polynucleotide, but is not limited thereto.

In the present disclosure, a polynucleotide salt may be, for example, polynucleotide sodium, polynucleotide potassium, polynucleotide calcium, polynucleotide magnesium, polynucleotide zinc, and the like. In addition, a polydeoxyribonucleotide salt may be, for example, polydeoxyribonucleotide sodium, polydeoxyribonucleotide potassium, polydeoxyribonucleotide calcium, polydeoxyribonucleotide magnesium, polydeoxyribonucleotide zinc, and the like. However, the polynucleotide salt and the polydeoxyribonucleotide salt are not limited thereto. According to embodiments of the present disclosure, the polynucleotide salt may be polynucleotide sodium, but is not limited thereto.

In the present disclosure, the DNA fraction may have a molecular weight of 1,800 kDa to 6,000 kDa, preferably in the range of 1,800 kDa to 2,300 kDa, and more preferably in the range of 2,000 kDa to 2,300 kDa. However, the molecular weight of the DNA fraction is not limited thereto.

When the molecular weight of the DNA fraction is less than the above range, the viscoelasticity of the finally prepared filler may be extremely low, and the filler may fail to function as a physical support. When the molecular weight of the DNA faction exceeds the above range, the molecular weight itself may cause problems, such as preparation speed, filter blockage, and the like, in the application of the sterile filter, thereby reducing the preparation efficiency.

In the present disclosure, the DNA fraction may be mixed to be included at a concentration in a range of 0.5% (w/v) to 2% (w/v) and preferably in the range of 1% (w/v) to 1.5% (w/v), with respect to the total volume of the final filler, but is not limited thereto.

When mixing the DNA mixture below the above range, the effects of the DNA fraction, for example, skin regeneration, increasing skin density, stimulating collagen production, and the like, may be marginal. In addition, the viscoelasticity required as physical support may be poor. When including the DNA mixture in excess of the above range, the increased viscoelasticity of the prepared filler may cause pain when injected into the skin, giving low satisfaction to patients and also making practitioners uncomfortable.

Any solvent capable of dissolving the DNA fraction while not causing a decrease in the preparation efficiency in the application of the sterile filter may be used as the solvent in the a) mixing step of the present disclosure. However, according to embodiments of the present disclosure, the solvent is preferably water for injection.

In the experimental example to be described later, it was specifically confirmed that when using water for injection as the solvent, the speed of bacteria removal in the b) removing step, to which the sterile filter is applied, was fast, and the blockage of the sterile filter was able to be prevented, thereby significantly improving the preparation efficiency.

In the present disclosure, the a) mixing step may further include stirring the solution after mixing the DNA fraction with the solvent so that the DNA fraction can be evenly (or homogeneously) distributed in the solvent.

The stirring may increase the speed of bacteria removal with the use of the sterile filter in the b) removing step. In addition, filter blockage caused by the aggregation of the DNA fraction can be prevented, resulting in an increase in the preparation efficiency.

In the present disclosure, the b) removing step is a step of removing the bacteria from the produced solution, in which the DNA fraction is dissolved, using the sterile filter.

In the present disclosure, the "sterile filter", used during a bioprocess, or filling and formulation processes, means a filter mainly made of PES, PVDF, nylon, or PTFE as a raw material.

In the present disclosure, the sterile filter may have a pore size suitable for removing harmful bacteria. According to embodiments of the present disclosure, the sterile filter may have a pore size of 0.2 µm, but is not limited thereto.

In the present disclosure, the order of introducing the b) removing step, to which the sterile filter is applied, is important for the integrity of the prepared filler and the preparation efficiency. That is, for the purpose of the present disclosure, the b) removing step is necessarily required to be performed before mixing the hyaluronic acid and/or pH modifier. Rather than applying each sterile filter to the solution in which the DNA fraction is dissolved and the solution in which the hyaluronic acid and/or pH modifier are dissolved, and then mixing the solutions, mixing the hyaluronic acid and/or pH modifier after applying the sterile filter to the solution in which the DNA fraction is dissolved is advantageous in terms of filtering speed.

In the present disclosure, the c) mixing step is a step of mixing the hyaluronic acid and/or pH modifier with the bacteria-removed solution.

In the present disclosure, the "hyaluronic acid" is a biopolymeric material, a linear polysaccharide composed of glucuronic acid and acetylglucosamine, which is one of the glycosaminoglycans present in extracellular matrix (ECM), synovial fluid of joint, and scaffolds for cartilage. Hyaluronic acid has viscoelastic properties, and thus can be used as a skin supplement and the like. In addition, hyaluronic acid is a material having excellent biocompatibility and has no problem in terms of immunity when applied in vivo.

In the present disclosure, the meaning of the hyaluronic acid encompasses all hyaluronic acid itself, a salt thereof, or a combination thereof. In addition, the hyaluronic acid may be directly synthesized or purchased from a commercially available product for use.

Hyaluronic acid salt may be, for example, sodium hyaluronate, potassium hyaluronate, calcium hyaluronate, magnesium hyaluronate, zinc hyaluronate, or cobalt hyaluronate, but is not limited thereto. According to embodiments of the present disclosure, the hyaluronic acid salt may be sodium hyaluronate, but is not limited thereto.

In the present disclosure, the hyaluronic acid may have a molecular weight in a range of 100 kDa to 5,000 kDa, preferably in the range of 500 kDa to 1,200 kDa and more preferably in the range of 700 kDa to 1,200 kDa.

When the molecular weight of the hyaluronic acid is lower than the above range, the viscosity of the finally prepared filler may be extremely low, and the filler may fail to properly function as a physical support. When the molecular weight of the hyaluronic acid exceeds the above range, the viscosity of the finally prepared filler may be extremely high, which is unsuitable for the intended use of the present disclosure, for example, use for reducing mild wrinkles, and may cause pain when injected into the skin.

In the present disclosure, the hyaluronic acid may be non-crosslinked (or uncrosslinked).

In the case of existing fillers, a cross-linking agent is used to prevent the degradation of hyaluronic acid caused by a bio-enzyme and to enhance elasticity. The cross-linking agent is mainly prepared using chemical cross-linking agents, such as 1,4-butanediol diglycidyl ether (BDDE), divinyl sulfone (DVS), and the like. Due to the side effects and safety issues of such chemical cross-linking agents, a washing step is essential in the preparation process. In addition, it is problematic that the residual amount of the cross-linking agent is required to be checked, resulting in a decrease in the efficiency of the preparation process.

In the filler preparation of the present disclosure, the non-crosslinked hyaluronic acid is used, so the washing step or the step of checking the residual amount of the cross-linking agent, necessarily required in the case of using the cross-linking agent, is unrequired. Thus, the efficiency of the preparation process can be maximized. In addition, despite using the non-crosslinked hyaluronic acid, the filler prepared by the preparation method of the present disclosure can obtain an in vivo degradation rate not inferior to that of existing products. As a result, the filler of the present disclosure can have a longer duration of tissue repair and achieve better collagen formation than existing products, thereby making good use thereof as a filler for in vivo injection.

In the present disclosure, the hyaluronic acid may be mixed to be included at a concentration in a range of 0.1% (w/v) to 1% (w/v) with respect to the total volume of the final filler.

When the concentration of the hyaluronic acid is lower than the above range, the viscosity of the finally prepared filler may be extremely low, and the filler may fail to function as a physical support. When the concentration of the hyaluronic acid exceeds the above range, the viscosity of the finally prepared filler may be extremely high, causing pain when injected into the skin. In addition, the filler may be unsuitable for the intended use of the present disclosure, for example, use for reducing mild wrinkles.

In the present disclosure, the "pH modifier" means a material used for regulating or stabilizing pH.

The pH modifier may be, for example, sodium chloride, sodium monohydrogen phosphate, sodium dihydrogen phosphate, or a mixture thereof, but is not limited thereto.

In the present disclosure, the pH modifier may include one in a concentrated form dissolved in the solvent, such as water for injection and the like. That is, in the present disclosure, the pH modifier itself may be mixed with the bacteria-removed solution in the b) removing step. Alternatively, the pH modifier may be dissolved in the solvent, such as water for injection and the like, concentrated, and then mixed with the bacteria-removed solution in the b) removing step.

In the c) mixing step of the present disclosure, at least one selected from among the hyaluronic acid and pH modifier may be mixed. For example, in the c) mixing step, the hyaluronic acid or pH modifier may be mixed, or both the hyaluronic acid and pH modifier may be mixed.

In the case of mixing both the hyaluronic acid and pH modifier in the c) mixing step of the present disclosure, the hyaluronic acid and pH modifier are mixed in a stepwise manner. Alternatively, the hyaluronic acid and pH modifier may be first mixed and then mixed with the bacteria-removed solution in the b) removing step.

According to embodiments of the present disclosure, in the case of mixing the hyaluronic acid and pH modifier in a stepwise manner, the c) mixing step may include: c-1) step of mixing the hyaluronic acid in the bacteria-removed solution produced in the b) removing step, to obtain a mixture; and c-2) step of mixing the pH modifier in the obtained mixture.

In the case of mixing the pH modifier in the c) mixing step of the present disclosure, the pH modifier may be mixed in an amount that allows the prepared filler to have a pH in a range of 6.0 to 8.0.

In the present disclosure, as the steps a) to c) are sequentially performed, a drying step is not required, unlike in the case of using a conventional method in which a bacteria-removed solution is dried and then re-dissolved to prepare a fraction. As a result, the efficiency of the preparation process can be maximized while maintaining sterile conditions by minimizing the possibility of contamination from the outside as per the reduced number of steps.

The preparation method of the present disclosure may further include mixing an anesthetic agent, and the anesthetic agent is preferably mixed after the c) mixing step.

In the present disclosure, the term "anesthetic agent" means a component used to relive itchiness and pain caused by skin inflammation. Examples of the anesthetic agent may include procaine, chloroprocaine, tetracaine, prilocaine, lidocaine, mepivacaine, bupivacaine, ropivacaine, etidocaine, lidocaine hydrochloride, benzocaine, oxetacaine, and the like. According to one embodiment of the present disclosure, the anesthetic agent is preferably lidocaine or a salt thereof (for example, lidocaine hydrochloride monohydrate), but is not limited thereto.

In the present disclosure, lidocaine, used for local anesthesia, may also have an effect of improving the elasticity of fibrous tissue in a combination with the polynucleotide and/or hyaluronic acid.

In the present disclosure, the anesthetic agent may be mixed to be included at a concentration in a range of 0.2% (w/v) to 0.4% (w/v) with respect to the total volume of the final filler. According to embodiments of the present disclosure, the anesthetic agent may be mixed to be included at 0.3% (w/v). When including the anesthetic agent below the above range, the anesthetic effect may be poor. When including the anesthetic agent in excess of the above range, the anesthetic effect may last long, and patients thus may have difficulty quickly returning to their daily lives through a quick and simple procedure. In addition, the toxicity may cause side effects.

According to another aspect of the present disclosure, a filler prepared by the preparation method of the present disclosure is provided.

In the present disclosure, the filler may include the DNA fraction at a concentration in a range of 0.5% (w/v) to 2% (w/v) and the hyaluronic acid at a concentration in a range of 0.1% (w/v) to 1% (w/v), with respect to the total volume of the final filler.

When the filler prepared by the preparation method of the present disclosure includes the DNA fraction and hyaluronic acid at concentrations of lower than the above ranges, the viscosity of the finally prepared filler may be extremely low, and the filler may fail to function as a physical function. When the filler prepared by the preparation method of the present disclosure includes the DNA fraction and hyaluronic acid at concentrations exceeding the above ranges, the viscosity of the finally prepared filler may be extremely high, causing pain when injected into the skin. In addition, the filler may be unsuitable for the intended use of the present disclosure, for example, use for reducing mild wrinkles.

In the present disclosure, the filler may have a complex viscosity in a range of 0.3 Pa·s to 3 Pa·s, preferably in the range of 1 Pa·s to 3.0 Pa·s, at a temperature of 25°C when being measured with a rheometer. According to embodiments of the present disclosure, the complex viscosity may be in a range of 1 Pa·s to 1.5 Pa·s, but is not limited thereto.

In the present disclosure, the filler may have a storage modulus G' of 5 Pa or higher at a temperature of 25°C when being measured with a rheometer.

In the present disclosure, the "complex viscosity (η*)" is the result of a complex interplay between viscosity and elasticity and means a mathematical expression represented by the sum of real and imaginary numbers.

In the present disclosure, the "storage modulus", related to elasticity, means a measure of stored energy, indicating that the higher the storage modulus, the greater the force to return to its original state.

Despite the low amounts of the DNA fraction and hyaluronic acid, the filler prepared by the preparation method of the present disclosure can obtain complex viscosity and storage modulus suitable for the intended use of the present disclosure.

In the present disclosure, the filler may be used for tissue repair. Specifically, the filler may be used for reducing mild wrinkles or facial contouring, and is more specifically for reducing mild wrinkles.

Wrinkles, evaluated in clinical trials according to the Wrinkle Severity Rating Scale (WSRS), may be divided into 5 grades, which are absent, mild, moderate, severe, and extreme.

Conventional botulinum toxin alone has therapeutic limitations in treating mild wrinkles. In addition, due to the viscosity of existing filler products, direct administration to the dermal layer, which plays a key role in collagen production, has limitations. That is, obtaining suitable complex viscosity and storage modulus is important to be used as dermal fillers for injection. In particular, fillers used for wrinkles are required to have an appropriate complex viscosity according to wrinkle conditions, divided from mild to severe wrinkles.

The filler prepared by the preparation method of the present disclosure exhibits a complex viscosity in a range of 0.3 Pa·s to 3.0 Pa·s, and can be thus effectively used for reducing mild wrinkles. In addition, compared to fillers injected into the entire face, the filler of the present disclosure exhibits a low injection pressure, causing low pain even when applied to thin skin, such as the eye area. According to the experimental examples to be described later, it was confirmed that despite the low amount of the DNA fraction compared to that of conventionally available filler products, the filler of the present disclosure is superior in terms of skin regeneration, collagen formation ability, and the like, and a natural look, compared to that when using the existing filler products, can be ensured.

In the present disclosure, the filler may be used for reducing crow's feet. The skin tissue around the eyes is extremely thin, thereby sensitively responding to microstimulation, and corresponds to the area where wrinkles are likely to be formed. Typically, wrinkles around the eye area include wrinkles in the form of crow's feet formed on the outer corners of the eyes and wrinkles under the eyes. The filler of the present disclosure has a relatively low viscosity and thus can be effectively applied to mild wrinkles formed on thin skin tissue such as the eye area.

A subject may be administered the filler of the present disclosure in an effective amount by subcutaneous injection, intradermal injection, and the like.

In the present disclosure, the "effective amount" means an amount of the filler sufficient to exhibit beneficial cosmetic or therapeutic effects of filler injection. The level of the effective amount may be determined by an operator according to factors including age, sex, sensitivity, injection time, wrinkle condition, and the like of the subject, as well as other factors well-known in the medical field.

In addition, filler injections may be performed, for example, once, multiple times, and/or over one or more extended periods of time.

In the present disclosure, the "subject" means an individual or patient in need of treatment for a specific condition or disease. The subject may be a mammal, and is preferably a human.

In the present disclosure, the filler may be a mesofiller injected through mesotherapy.

In the present disclosure, the "mesotherapy", in which a filler is directly injected into a target area, means an injection therapy aiming for minimizing side effects, for improving effectiveness, and for prolonging duration by injecting the filler in an extremely small amount into a middle layer of skin (skin dermal layer) using a microsyringe.

In the present disclosure, the "mesofiller", a compound word for mesotherapy and filler, is used for the purpose of smoothing wrinkles by injecting a filler between a skin dermal layer and fibrous tissue, and may exhibit effects of regenerating skin, improving elasticity, and reducing fine lines.

According to a further aspect of the present disclosure, a pre-filled syringe filled with the filler prepared by the preparation method of the present disclosure is provided.

Unless otherwise specified, the detailed description related to the filler and the pre-filled syringe herein is the same as those mentioned in the preparation method of the filler. Thus, a separate description will be omitted to avoid excess complicity of the specification.

### Advantageous Effects

In a filler preparation method of the present disclosure, a filler prepared thereby can obtain the viscoelasticity required as a physical support.

In addition, in the filler preparation method of the present disclosure, a change in the content of the DNA fraction can be prevented in a bacteria-removing step. Thus, despite the low content of the DNA fraction compared to that of existing products, the prepared filler has excellent effects of improving skin thickness and reducing skin wrinkles, collagen formation ability, and the like.

Furthermore, in the filler preparation method of the present disclosure, the low content of the DNA fraction is used, so the prepared filler causes low pain compared to currently available fillers, and thus gives satisfaction to patients while making practitioners comfortable.

Moreover, in the filler preparation method of the present disclosure, non-crosslinked hyaluronic acid is used, so steps, such as a washing step for removing a cross-linking agent, a step of checking the residual amount of the cross-linking agent, and the like, are unnecessary. As a result, the efficiency of the preparation process is excellent, and the stability of the prepared filler is excellent because no inflammatory responses are found according to the use of the cross-linking agent.

### Description of Drawings

FIG. 1 shows a graph comparing the in vivo degradation rates of a filler sample of Example 3 and a product from other manufacturers in Experimental Example 7; and
FIG. 2 shows images of filler samples of Example 2 and Comparative Examples 4 to 6 in Experimental Example 8, in which FIG. 2(a) shows an image immediately after preparation, and FIG. 2(b) shows an image after leaving the filler samples at a temperature of 35°C for 2 weeks.

### Best Mode

Hereinafter, the present disclosure will be described in detail with reference to embodiments. However, the embodiments of the present disclosure are disclosed only for illustrative purposes and should not be construed as limiting the present disclosure. In addition, unless otherwise defined, all terms used herein have the same meaning as commonly understood in the art to which the present disclosure belongs.

### Example 1 and Comparative Examples 1 to 3

6.25 g of polynucleotide sodium (manufacturer: HTL, molecular weight: 1,800 kDa to 2,300 kDa) was dissolved in water for injection or phosphate-buffered saline to produce each solution containing polynucleotide sodium at a concentration of 1.25% (w/v). Each of the solutions was set to have a volume of 500 mL and a pH in a range of 6.0 to 8.0. Thereafter, each of the dissolved solutions was unprocessed, or processed by using wet sterilization (at a temperature of 121°C for 20 minutes) or a sterile filter (0.2 µm, PES) to prepare fillers.

Solvents and process methods used in each of Example and Comparative Examples are shown in Table 1 below.

**[Table 1]**

| | **Solvent** | **Process method** |
|---|---|---|
| **Example 1** | Water for injection | Sterile filter |
| **Comparative Example 1** | Water for injection | Unprocessed |
| **Comparative Example 2** | Water for injection | Wet sterilization |
| **Comparative Example 3** | Phosphate-buffered saline | Sterile filter |

### Experimental Example 1. Analysis of rheological properties

A rheometer (KNX2210, MALVERN, U.K.) was used to analyze the rheological properties of the fillers prepared according to the solvents and process methods. The results thereof are shown in Table 2 below.

A filler for tissue repair functions as a physical support after injected into the skin, so storage modulus (G') and complex viscosity (η*) values were regarded as the most important factors.

**[Table 2]**

| | **G* (Pa)** | **G' (Pa)** | **G" (Pa)** | **η* (Pa·s)** | **δ (°)** |
|---|---|---|---|---|---|
| **Example 1** | 3.39305 | 1.3815 | 3.099 | 0.54005 | 65.975 |
| **Comparative Example 2** | 0.08717 | 0.086865 | 0.0071915 | 0.01387 | -4.805 |
| **Comparative Example 3** | 7.8095 | 6.3585 | 4.5345 | 1.243 | 35.49 |

As shown in Table 2 above, the filler of Comparative Example 2 using the wet sterilization method exhibited a significantly lower complex viscosity than the filler of Example 1 using the sterile filter in the preparation method, confirming that the filler of Comparative Example 2 was inappropriate to be used as the filler for tissue repair required to function as the physical support after injected into the skin.

In addition, in the case of Comparative Example 3 using phosphate-buffered saline as the solvent, the storage modulus and complex viscosity were extremely high. In particular, the filtering speed was extremely slow in the process of bacteria removal, and filter blockage occurred rapidly. For this reason, the filler of Comparative Example 3 was inappropriate for use in the filler preparation method of the present disclosure.

### Experimental Example 2. Measurement of polynucleotide sodium content

To confirm a change in the content of polynucleotide sodium according to the process methods performed on the prepared fillers, HPLC was used to measure the content thereof. The results thereof are shown in Table 3 below.

**[Table 3]**

| | **Content of polynucleotide sodium (%)** |
|---|---|
| **Example 1** | 107.72 |
| **Comparative Example 1** | 106.73 |
| **Comparative Example 2** | 94.87 |

As shown in Table 3 above, the filler of Example 1 using the sterile filter in the preparation method exhibited the content of polynucleotide sodium not inferior to that of the unprocessed group, confirming that the method of using the sterile filter hardly affected the content of polynucleotide sodium. On the other hand, a significant decrease in the content of polynucleotide sodium was confirmed in Comparative Example 2 using the wet sterilization method.

### Example 2 and Comparative Examples 4 to 6

### (1) Preparation of Example 2

6.25 g of polynucleotide sodium (manufacturer: HTL, molecular weight: 1,800 kDa to 2,300 kDa) was dissolved in water for injection, and the dissolved solution was then preprocessed using a sterile filter (0.2 µm, PES).

Next, non-crosslinked sodium hyaluronate (molecular weight: 700 kDa to 1,200 kDa) was completely dissolved in the solution, and concentrated phosphate-buffered saline was then added thereto so that the resulting solution had a volume of 500 mL and a pH in a range of 6.0 to 8.0. As a result, a filler of Example 2 containing polynucleotide sodium and non-crosslinked sodium hyaluronate at final concentrations of 10 mg/ml and 3 mg/ml, respectively, was prepared.

### (2) Preparation of Comparative Examples 4 to 6

Polynucleotide sodium (manufacturer: HTL, molecular weight: 1,800 kDa to 2,300 kDa) and non-crosslinked sodium hyaluronate (molecular weight: 700 kDa to 1,200 kDa) were mixed with phosphate-buffered saline and dissolved to prepare a filler of Comparative Example 4.

In addition, polynucleotide sodium (manufacturer: HTL, molecular weight: 1,800 kDa to 2,300 kDa) and non-cross-linked sodium hyaluronate (molecular weight: 700 kDa to 1,200 kDa) were mixed with phosphate-buffered saline, and dissolved. Then, using wet sterilization or a sterile filter (0.2 µm, PES) for bacteria removal, respectively, a filler of Comparative Example 5 (wet-sterilized) and a filler of Comparative Example 6 (bacteria-removed) were each independently prepared.

Each of the fillers of Comparative Examples 4 to 6 was set to contain polynucleotide sodium and non-crosslinked sodium hyaluronate at final concentrations of 10 mg/ml and 3 mg/ml, respectively.

### Experimental Example 3. Analysis of rheological properties

A rheometer (KNX2210, MALVERN, U.K.) was used to analyze the rheological properties according to the preparation methods of the prepared fillers. The results thereof are shown in Table 4 below.

**[Table 4]**

| | **G* (Pa)** | **G' (Pa)** | **G" (Pa)** | **η* (Pa·s)** | **δ (°)** |
|---|---|---|---|---|---|
| **Example 2** | 8.094 | 5.712 | 5.734 | 1.288 | 45.11 |
| **Comparative Example 5** | 0.1084 | 0.07653 | 0.07679 | 0.01725 | 45.1 |
| **Comparative Example 6** | 6.682 | 4.365 | 5.0585 | 1.0635 | 49.22 |

As shown in Table 4 above, in the case of the fillers of Comparative Examples 5 and 6 using the wet sterilization and the sterile filter, respectively, after simultaneously dissolving the polynucleotide sodium and the non-crosslinked sodium hyaluronate, viscoelastic properties were deteriorated compared that of the filler of Example 2 prepared stepwise process after dissolving polynucleotide sodium in water for injection. In particular, the filler of Comparative 5 prepared by the sterilization exhibited a significantly low complex viscosity, confirming that the filler of Comparative 5 was inappropriate to be used as the filler for tissue repair required to function as a physical support after injected into the skin.

In addition, in the case of Comparative Example 6, the filtering speed was significantly low in the process of bacteria removal, and filter blockage occurred rapidly, confirming that there was a problem in the preparation efficiency.

### Experimental Example 4. Confirmation of polynucleotide sodium content

To confirm a change in the contents of the main component according to the preparation methods of the prepared fillers, HPLC was used to measure the content of polynucleotide sodium. The results thereof are shown in Table 5 below.

**[Table 5]**

| | **Content of polynucleotide sodium (%)** |
|---|---|
| **Example 2** | 106.92 |
| **Comparative Example 4** | 107.02 |
| **Comparative Example 5** | 101.23 |
| **Comparative Example 6** | 104.04 |

As shown in Table 5 below, the filler of Example 2 prepared by the preparation method of the present disclosure exhibited the content of polynucleotide sodium not inferior to that of Comparative Example 4 not involving separate bacteria removal after the simple mixing, which hardly affected the content of polynucleotide sodium. On the other hand, in the case of Comparative Examples 5 and 6 involving the sterilization and the bacteria removal, respectively, after mixing polynucleotide sodium and non-crosslinked sodium hyaluronate, a decrease in the content of polynucleotide sodium was confirmed.

This means that, in the preparation, the content of the polynucleotide sodium, an active ingredient contained in the final filler, may vary according to process order.

### Experimental Example 5. Measurement of injection pressure

Whether the filler prepared by the preparation method of the present disclosure had high adaptability to a patient when used for injection was examined by the analysis of injection pressure (injection force). The results thereof are shown in Table 6 below.

The injection pressure was measured using a tensile strength tester (JSV H1000, JISC, JAPAN) with a 33-G injection needle at test speeds of 5 mm/min and 30 mm/min.

**[Table 6]**

| | **5 mm/min** | **30 mm/min** |
|---|---|---|
| **Example 2** | 4.19 N | 10.38 N |

As shown in Table 6 above, the filler prepared by the preparation method of the present disclosure was confirmed to have a low injection pressure. This confirms that the filler causes less pain when injected into patients, and makes practitioners more comfortable.

### Example 3

6.25 g of polynucleotide sodium (manufacturer: HTL, molecular weight: 1,800 kDa to 2,300 kDa) was completely dissolved in water for injection and then filtered for bacterial removal. Next, non-crosslinked sodium hyaluronate (molecular weight: 700 kDa to 1,200 kDa) and concentrated phosphate-buffered saline were added thereto so that the resulting solution had a volume of 500 mL and a pH in a range of 6.0 to 8.0. As a result, a filler of Example 3 containing polynucleotide sodium and non-crosslinked sodium hyaluronate at final concentrations of 10 mg/ml and 3 mg/ml, respectively, was prepared.

### Experimental Example 6. Evaluation of wrinkle reduction and collagen production

In Experimental Example 6, a comparative evaluation of wrinkle reduction and collagen production was performed through animal efficacy evaluation.

Specifically, the prepared filler of Example 3 was injected into an SKH-1-Hairless mouse. Then, the volume of an injection site was measured for a month while performing histopathology evaluation. As a control group, a polynucleotide filler product (with the content of polynucleotide sodium of 2%) currently on sale was used.

The results thereof are shown in Table 7 below.

**[Table 7]**

| | **Total Skin Thickness* (µm)** | **Epidermal Thickness (µm)** | **Epidermal Microfolds (microfold/mm)** | **Collagen Fibers (%/mm²)** |
|---|---|---|---|---|
| **Example 3** | 4.93 | 24.89 | 15.6 | 10.80 |
| **Product from other manufacturers** | 4.76 | 21.55 | 17.55 | 6.81 |

| | | | | |
|---|---|---|---|---|
| (* Total Skin Thickness is described at a scale of 1/100) | | | | |

As confirmed in Table 7 above, the filler prepared by the preparation method of the present disclosure was confirmed to have superior effects of improving skin thickness and reducing skin wrinkles as well as forming collagen to the product from other manufacturers.

On the other hand, no remnant materials were found in the filler of Example 3 prepared by the preparation method of the present disclosure, after the injection. In addition, foreign body reactions and adverse reactions caused by the injection of Example 3 were hardly observed during the test period (1 month), confirming that the stability was also good.

### Experimental Example 7. Evaluation of in vivo degradation rate and volume change

In Experimental Example 7, a comparative evaluation of the in vivo degradation rates of the filler prepared by the preparation method of the present disclosure and an existing filler was performed.

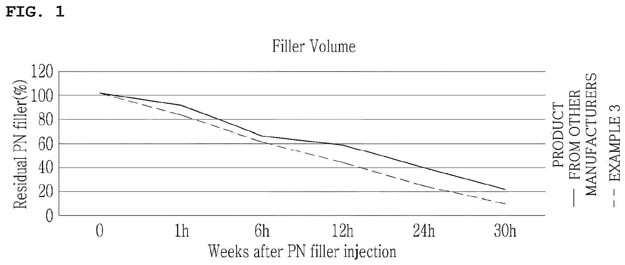

Specifically, the prepared filler of Example 3 was injected into an SKH-1-Hairless mouse, and a volume change of an injection solution was observed for 30 hours. As a control group, a polynucleotide filler product (with the content of polynucleotide sodium of 2%) currently on sale was used. The results thereof are shown in FIG. 1.

As confirmed in FIG 1, the time to reach complete degradation was predicted using a trendline after the observation for 30 hours. As a result, the predicted time to reach complete degradation was about 34 hours in Example 3. Even though the concentration of polynucleotide was lower in the filler of Example 3 than that of the other product currently on sale in which the predicted time to reach complete degradation was about 37 hours, there was no significant difference in terms of the in vivo degradation rate.

In addition, when changes in the filler volume were observed in a macroscopic manner, using a folliscope and PRIMOS, in the case of injecting the filler product from other manufacturers, the volume became small while maintaining the injected shape. On the other hand, in the case of injecting the filler of Example 3, the volume became small while the injected shape spread in a further natural way to the sides, confirming that the filler of Example 3 had aesthetic stability suitable to be used as a skin injection filler.

### Experimental Example 8. Examination of effect according to difference in order of the mixing step of the polynucleotide and non-crosslinked hyaluronic acid and sterilization step

In Experimental Example 8, a comparative evaluation of the effect according to a difference in the order of the mixing step of the polynucleotide and non-crosslinked hyaluronic acid and the sterilization step, using the filler of Example 2 and the fillers of Comparative Examples 4 to 6.

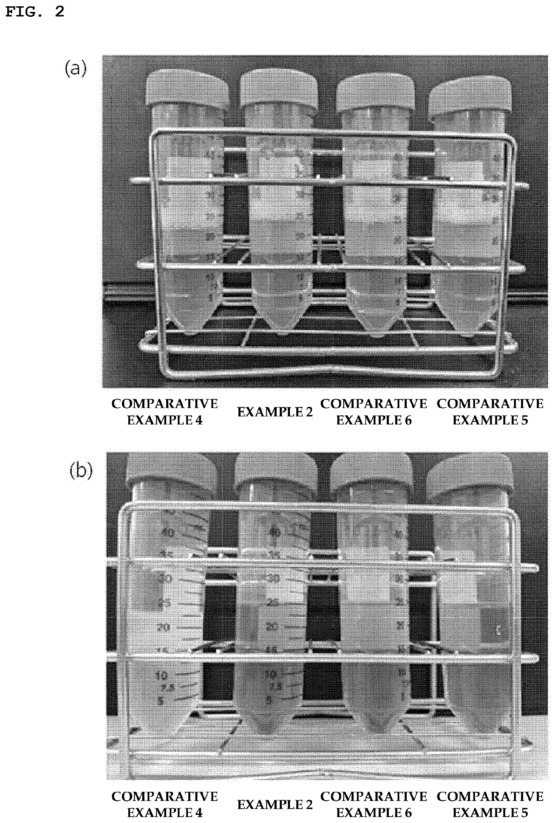

Specifically, whether the filler of Example 2 and the filler samples of Comparative Examples 4 to 6 were degenerated or not was examined through comparison immediately after the respective preparations and leaving each of the filler samples at a temperature of 35°C for 2 weeks. The results thereof are shown in FIG. 2.

As confirmed in FIG. 2 (b), the filler sample of Comparative Example 4 degenerated when two weeks elapsed. In addition, the filler samples of Example 2 and Comparative Examples 5 and 6 did not degenerate.

However, when comparing the preparation process, there was a delay in the filtering of the filler sample of Comparative Example 6, prepared by filtering the solution of the polynucleotide and non-crosslinked hyaluronic acid for bacteria removal, taking longer time in the preparation than that in the preparation of the filler of Example 2. In the case of the filler sample of Comparative Example 5, the wet sterilization was required to be performed at a temperature of 121°C for 20 minutes, taking longer time in the preparation than that in the preparation of the filler of Example 2.

Thus, the filler sample of Example 2, prepared by quickly filtering the polynucleotide solution for bacteria removal and then mixing the solution with non-crosslinked hyaluronic acid, was confirmed to shorten the preparation time while obtaining excellent integrity, compared to the filler samples of Comparative Examples 5 and 6.

## Claims

1. A method of preparing a filler, the method comprising:
a) mixing a DNA fraction with a solvent to produce a solution;
b) removing bacteria from the produced solution using a sterile filter; and
c) mixing hyaluronic acid and a pH modifier with the bacteria-removed solution.

2. The method of claim 1, wherein the DNA fraction is polynucleotide (PN), polydeoxyribonucleotide (PDRN), or a mixture thereof.

3. The method of claim 1, wherein the DNA fraction has a molecular weight in a range of 1,800 kDa to 6,000 kDa.

4. The method of claim 1, wherein the DNA fraction is comprised at a concentration in a range of 0.5% (w/v) to 2% (w/v) with respect to the total volume of the final filler.

5. The method of claim 1, wherein the solvent is water for injection.

6. The method of claim 1, further comprising stirring the solution after the mixing of the solvent in the a) mixing of the DNA fraction.

7. The method of claim 6, wherein the DNA fraction is evenly distributed in the solvent.

8. The method of claim 1, wherein the sterile filter has a pore size of 0.2 µm.

9. The method of claim 1, wherein the hyaluronic acid is non-crosslinked.

10. The method of claim 1, wherein the hyaluronic acid has a molecular weight in a range of 100 kDa to 5,000 kDa.

11. The method of claim 1, wherein the hyaluronic acid is contained at a concentration in a range of 0.1% (w/v) to 1% (w/v) with respect to the total volume of the final filler.

12. The method of claim 1, wherein the c) mixing of the hyaluronic acid and pH modifier comprises:
c-1) mixing the hyaluronic acid in the bacteria-removed solution produced in the b) removing, to obtain a mixture; and
c-2) mixing the pH modifier in the obtained mixture.

13. The method of claim 1, further comprising mixing an anesthetic agent after the c) mixing of the hyaluronic acid and pH modifier.

14. The method of claim 13, wherein the anesthetic agent is lidocaine or a salt thereof.

15. The method of claim 13, wherein the anesthetic agent is comprised at a concentration of 0.3% (w/v) with respect to the total volume of the final filler.

16. A filler prepared by the method of any one of claims 1 to 15.

17. The filler of claim 16, wherein the filler has a complex viscosity in a range of 0.3 Pa·s to 3 Pa·s when being measured with a rheometer.

18. The filler of claim 16, wherein the filler is used for tissue repair.

19. The method of claim 16, wherein the filler is used for reducing mild wrinkles or for facial contouring.

20. A pre-filled syringe filled with the filler of claim 16.
